(19) Europäisches Patentamt / European Patent Office / Office européen des brevets

(11) **EP 3 405 899 B1**

(12) **FASCICULE DE BREVET EUROPEEN**

(45) Date de publication et mention
de la délivrance du brevet:
**11.08.2021 Bulletin 2021/32**

(21) Numéro de dépôt: **17705020.0**

(22) Date de dépôt: **23.01.2017**

(51) Int Cl.:
*G16B 40/00* (2019.01)     *C12Q 1/6827* (2018.01)

(86) Numéro de dépôt international:
**PCT/EP2017/051327**

(87) Numéro de publication internationale:
**WO 2017/125606 (27.07.2017 Gazette 2017/30)**

(54) **PROCÉDÉ DE CLASSIFICATION D'UN ÉCHANTILLON BIOLOGIQUE**

VERFAHREN ZUR KLASSIFIZIERUNG VON EINER BIOLOGISCHEN PROBE

PROCESS FOR CLASSIFYING A BIOLOGICAL SAMPLE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.01.2016 FR 1650527**

(43) Date de publication de la demande:
**28.11.2018 Bulletin 2018/48**

(73) Titulaires:
• **Université de Montpellier**
**34090 Montpellier (FR)**
• **Institut Recherche pour le Developpement**
**13002 Marseille 2 (FR)**
• **Centre National de la Recherche Scientifique**
**75794 Paris (FR)**

(72) Inventeurs:
• **AVARRE, Jean-Christophe**
**34980 Montferrier sur Lez (FR)**
• **REYNES, Christelle**
**34070 Montpellier (FR)**

(74) Mandataire: **IPAZ**
**10 boulevard Victor Hugo**
**34000 Montpellier (FR)**

(56) Documents cités:
**US-A1- 2013 218 476     US-A1- 2014 067 277**
**US-A1- 2015 232 926**

• **STEPHANIE I. FRALEY ET AL: "Nested Machine Learning Facilitates Increased Sequence Content for Large-Scale Automated High Resolution Melt Genotyping", SCIENTIFIC REPORTS, vol. 6, 18 janvier 2016 (2016-01-18), page 19218, XP055305851, DOI: 10.1038/srep19218**
• **SAMI KANDERIAN ET AL: "Automated Classification and Cluster Visualization of Genotypes Derived from High Resolution Melt Curves", PLOS ONE, vol. 10, no. 11, 25 novembre 2015 (2015-11-25), page e0143295, XP055305853, DOI: 10.1371/journal.pone.0143295**
• **ABHIJIT DASGUPTA ET AL: "Brief review of regression-based and machine learning methods in genetic epidemiology: the Genetic Analysis Workshop 17 experience", GENETIC EPIDEMIOLOGY, vol. 35, no. S1, 1 janvier 2011 (2011-01-01), pages S5-S11, XP055305965, US ISSN: 0741-0395, DOI: 10.1002/gepi.20642**
• **ROBERT PALAIS ET AL: "Chapter 13 Mathematical Algorithms for High-Resolution DNA Melting Analysis", METHODS IN ENZYMOLOGY, ACADEMIC PRESS, US, vol. 454, 1 janvier 2009 (2009-01-01), pages 323-343, XP009185077, ISSN: 0076-6879**
• **R. ISSA ET AL: "High resolution melting analysis for the differentiation of Mycobacterium species", JOURNAL OF MEDICAL MICROBIOLOGY., vol. 63, no. Pt_10, 18 juillet 2014 (2014-07-18), pages 1284-1287, XP055306051, GB ISSN: 0022-2615, DOI: 10.1099/jmm.0.072611-0**

**EP 3 405 899 B1**

- **Andy Liaw ET AL: "Classification and Regression by randomForest", R News, 1 décembre 2002 (2002-12-01), pages 18-22, XP055305332, Extrait de l'Internet: URL:http://www.bios.unc.edu/~dzeng/BIOS740 /randomforest.pdf [extrait le 2016-09-26] cité dans la demande**

- **Stefan Rödiger ET AL: "Surface Melting Curve Analysis with R", , 1 décembre 2013 (2013-12-01), XP055361909, Extrait de l'Internet: URL:https://journal.r-project.org/archive/ 2013-2/roediger-bohm-schimke.pdf [extrait le 2017-04-05]**

**Description**

**Domaine technique**

La présente invention concerne un procédé de classification d'un échantillon biologique, et un dispositif associé.
Un tel dispositif permet à un utilisateur de classifier un échantillon biologique parmi plusieurs groupes possibles. Le domaine de l'invention est celui de la classification biologique.

**Etat de la technique antérieure**

L'article « Surface Melting Curve Analysis with R » de Rödiger et al. (The R Journal. Vol. 5/2, décembre 2013) décrit une méthode de fabrication d'un « résumé » ou d'une description d'une courbe de fusion.
On connaît le document WO2013/166373, qui décrit un procédé de la détermination du statut de régulation de la voie de signalisation IL-6/STAT3 dans un échantillon cellulaire ou chez un sujet. Le statut de régulation de la voie de signalisation IL-6/STAT3 dans un échantillon cellulaire ou chez un sujet peut être analysé sur la base du niveau d'expression d'un ou plusieurs parmi 16 gènes d'une signature d'expression. L'expression de biomarqueurs est de préférence déterminée par RT-PCR à l'aide de procédés SYBR Green, et des données d'expression sont analysées et comparées à un échantillon témoin par l'utilisation de la méthode à forêt aléatoire. La détermination des variables sélectionnées (ici les 16 gènes) est spécifique à la problématique et doit être manuellement réalisée pour chaque nouvelle problématique.
Certains problèmes techniques peuvent se poser pour un procédé de classification d'un échantillon biologique dans un groupe, notamment lorsque le nombre de groupes possibles est important, par exemple :

- Comment améliorer la finesse de la classification ? et/ou
- Comment être capable de traiter un échantillon qui n'appartient à aucun groupe connu ?
- Comment automatiser le procédé, sans sélection manuelle de variables ?

Le but de la présente invention est de résoudre au moins un de ces problèmes.

**Exposé de l'invention**

Cet objectif est atteint avec un procédé de classification d'un échantillon biologique de mesure, comprenant :

- une acquisition d'au moins une courbe de fusion d'ADN de l'échantillon biologique de mesure, dite au moins une courbe de mesure (de préférence une acquisition de plusieurs courbes de fusion d'ADN de l'échantillon biologique de mesure, aussi appelées réplicats techniques), typiquement chaque courbe de mesure comprenant différents points, chaque point correspondant à une grandeur proportionnelle ou représentative d'un taux ou d'une quantité de dénaturation de l'ADN de l'échantillon de mesure en fonction d'une température,
- une détermination d'une appartenance de l'échantillon biologique de mesure à un groupe déterminé parmi différents groupes possibles, par une analyse de descripteurs issus de l'au moins une courbe de mesure, chacune des étapes d'acquisition et de détermination impliquant l'utilisation d'un moyen technique.

caractérisé en ce que les descripteurs comprennent un ou plusieurs (de préférence plusieurs) point(s) de la dérivée seconde de chaque courbe de mesure, ledit ou chacun desdits points comprenant une valeur de la dérivée seconde de la courbe de fusion pour une valeur de température spécifique.
Les descripteurs peuvent comprendre en outre:

- un ou plusieurs (de préférence plusieurs) point(s) de la dérivée première de chaque courbe de mesure, et/ou

- un ou plusieurs point(s) de chaque courbe de mesure, et/ou
- un ou plusieurs percentile(s) de chaque courbe de mesure.

L'acquisition d'au moins une courbe de fusion d'ADN de l'échantillon biologique de mesure peut comprendre l'acquisition d'au moins une courbe de fusion d'un résultat d'une PCR obtenue en présence simultanée de plusieurs couples d'amorces ciblant plusieurs molécules cibles d'ADN, correspondants par exemple à plusieurs pathogènes. On parle alors de conditions de « multiplexage ». Ce mode de réalisation est utile pour accélérer les recherches, par exemple pour plusieurs pathogènes très rarement présents en commun dans un même échantillon biologique. Les cas rares, par exemple de plusieurs pathogènes présents en commun dans un échantillon, sont identifiés : typiquement, la courbe de fusion a autant de points d'inflexion que de pathogènes présents ; différents « groupes déterminés » peuvent alors réunir les différentes combinaisons de présence de ces différents pathogènes.

La détermination peut comprendre une détermination par une méthode des forêts aléatoires. Le procédé selon l'invention peut comprendre un apprentissage comprenant :

- une acquisition de différentes courbes de fusion d'ADN, dites courbes de référence, à partir de différents échantillons biologiques de référence appartenant à différents groupes initiaux connus et déterminés avant l'apprentissage, puis
- une détermination des descripteurs à partir des courbes de référence, puis
- une construction d'une forêt selon la méthode des forêts aléatoires, comprenant une construction de plusieurs arbres selon la méthode des forêts aléatoires, chaque arbre comprenant, à chaque nœud, l'utilisation d'un des descripteurs associé à un seuil et permettant de séparer les courbes de référence en deux sous-ensembles, chaque feuille de chaque arbre ne correspondant qu'à un seul groupe parmi les différents groupes possibles.

La détermination des descripteurs peut comprendre :

- une détermination préliminaire de descripteurs, puis
- une élimination de certains descripteurs redondants.

L'élimination de certains descripteurs peut comprendre, pour chaque ensemble de descripteurs exhibant deux à deux un coefficient de corrélation de Pearson supérieur à 0,95, une conservation d'un seul descripteur.

Le procédé selon l'invention peut comprendre :

- après l'acquisition de différentes courbes de référence (et/ou de préférence avant la construction des arbres), une identification de plusieurs courbes de référence correspondant au même groupe initial, dit groupe ambigu, et présentant des profils séparés en plusieurs sous-groupes, et
- une séparation de ce groupe ambigu en plusieurs groupes possibles.

Le procédé selon l'invention peut comprendre :

- après l'acquisition de différentes courbes de référence (et/ou de préférence après la construction des arbres), une identification de plusieurs courbes de référence correspondant à plusieurs groupes initiaux, dit groupes confondus, et présentant des profils réunis en un seul groupe, et
- une unification de ces groupes confondus en un unique groupe possible.

Le procédé selon l'invention peut comprendre en outre un calcul d'un indice de confiance de l'étape de détermination d'une appartenance de l'échantillon biologique de mesure à un groupe déterminé. Le calcul de l'indice de confiance peut comprendre :

- un calcul d'une distribution de proximités moyennes entre courbes de référence appartenant au groupe déterminé,
- un calcul d'une proximité moyenne de l'au moins une courbe de mesure avec les courbes de référence appartenant au groupe déterminé, et
- un calcul d'un taux de courbes de référence appartenant au groupe déterminé, et ayant une proximité moyenne aux autres courbes de référence appartenant au groupe déterminé inférieure à la proximité moyenne de l'au moins une courbe de mesure avec les courbes de référence appartenant au groupe déterminé.

Le procédé selon l'invention peut comprendre en outre, après l'étape de détermination d'une appartenance de l'échantillon biologique de mesure à un groupe déterminé, un refus d'affecter l'échantillon biologique de mesure à quelque groupe que ce soit en fonction de la valeur de l'indice de confiance.

Suivant encore un autre aspect de l'invention, il est proposé un dispositif de classification d'un échantillon biologique de mesure, comprenant :

- des moyens agencés et/ou programmés pour une acquisition d'au moins une courbe de fusion d'ADN de l'échantillon biologique de mesure, dite au moins une courbe de mesure (de préférence une acquisition de plusieurs courbes de fusion d'ADN de l'échantillon biologique de mesure, aussi appelées réplicats techniques), typiquement chaque courbe de mesure comprenant de préférence différents points, chaque point correspondant à une grandeur proportionnelle ou représentative d'un taux ou d'une quantité de dénaturation de l'ADN de l'échantillon de mesure en fonction d'une température,
- des moyens agencés et/ou programmés pour une détermination d'une appartenance de l'échantillon biologique de mesure à un groupe déterminé parmi différents groupes possibles, par une analyse de descripteurs issus de l'au moins une courbe de mesure,

caractérisé en ce que les descripteurs comprennent un ou plusieurs (de préférence plusieurs) point(s) de la dérivée seconde de chaque courbe de mesure, ledit ou chacun desdits points comprenant une valeur de la dérivée seconde de la courbe de fusion pour une valeur de température spécifique,

Les descripteurs peuvent comprendre en outre:

- un ou plusieurs (de préférence plusieurs) point(s) de la dérivée première de chaque courbe de mesure, et/ou
- un ou plusieurs point(s) de chaque courbe de mesure, et/ou
- un ou plusieurs percentile(s) de chaque courbe de mesure.

Les moyens agencés et/ou programmés pour la détermination comprennent de préférence des moyens agencés et/ou programmés pour une détermination par une méthode des forêts aléatoires. Le dispositif selon l'invention peut comprendre des moyens agencés et/ou programmés pour un apprentissage comprenant :

- des moyens agencés et/ou programmés pour une acquisition de différentes courbes de fusion d'ADN, dites courbes de référence, à partir de différents échantillons biologiques de référence appartenant à différents groupes initiaux connus et déterminés avant l'apprentissage, puis
- des moyens agencés et/ou programmés pour une détermination des descripteurs à partir des courbes de référence, puis
- des moyens agencés et/ou programmés pour une construction d'une forêt selon la méthode des forêts aléatoires, comprenant des moyens agencés et/ou programmés pour une construction de plusieurs arbres selon la méthode des forêts aléatoires, chaque arbre comprenant, à chaque nœud, l'utilisation d'un des descripteurs associé à un seuil et permettant de séparer les courbes de référence en deux sous-ensembles, chaque feuille de chaque arbre ne correspondant qu'à un seul groupe parmi les différents groupes possibles.

Les moyens agencés et/ou programmés pour la détermination des descripteurs peuvent comprendre :

- des moyens agencés et/ou programmés pour une détermination préliminaire de descripteurs,
- des moyens agencés et/ou programmés pour, après la détermination préliminaire, une élimination de certains descripteurs redondants.

Les moyens agencés et/ou programmés pour l'élimination de certains descripteurs peuvent comprendre des moyens agencés et/ou programmés pour, pour chaque ensemble de descripteurs exhibant deux à deux un coefficient de corrélation de Pearson supérieur à 0,95, une conservation d'un seul descripteur.

Le dispositif selon l'invention peut comprendre :

- des moyens agencés et/ou programmés pour, après l'acquisition de différentes courbes de référence (et/ou de préférence avant la construction des arbres), une identification de plusieurs courbes de référence correspondant au même groupe initial, dit groupe ambigu, et présentant des profils séparés en plusieurs sous-groupes, et
- des moyens agencés et/ou programmés pour une séparation de ce groupe ambigu en plusieurs groupes possibles.

Le dispositif selon l'invention peut comprendre :

- des moyens agencés et/ou programmés pour, après l'acquisition de différentes courbes de référence (et/ou de préférence après la construction des arbres), une identification de plusieurs courbes de référence correspondant à plusieurs groupes initiaux, dit groupes confondus, et présentant des profils réunis en un seul groupe, et
- des moyens agencés et/ou programmés pour une unification de ces groupes confondus en un unique groupe possible.

Le dispositif selon l'invention peut comprendre en outre des moyens agencés et/ou programmés pour un calcul d'un indice de confiance de l'étape de détermination d'une appartenance de l'échantillon biologique de mesure à un groupe déterminé. Les moyens agencés et/ou programmés pour le calcul de l'indice de confiance comprennent de préférence:

- des moyens agencés et/ou programmés pour un calcul d'une distribution de proximités moyennes entre courbes de référence appartenant au groupe déterminé,

- des moyens agencés et/ou programmés pour un calcul d'une proximité moyenne de l'au moins une courbe de mesure avec les courbes de référence appartenant au groupe déterminé, et
- des moyens agencés et/ou programmés pour un calcul d'un taux de courbes de référence appartenant au groupe déterminé, et ayant une proximité moyenne aux autres courbes de référence appartenant au groupe déterminé inférieure à la proximité moyenne de l'au moins une courbe de mesure avec les courbes de référence appartenant au groupe déterminé.

Le dispositif selon l'invention peut comprendre en outre des moyens agencés et/ou programmés pour, après l'étape de détermination d'une appartenance de l'échantillon biologique de mesure à un groupe déterminé, un refus d'affecter l'échantillon biologique de mesure à quelque groupe que ce soit en fonction de la valeur de l'indice de confiance.

**Description des figures et modes de réalisation**

D'autres avantages et particularités de l'invention apparaîtront à la lecture de la description détaillée de mises en œuvre et de modes de réalisation nullement limitatifs, et des dessins annexés suivants :

- la figure 1 est un organigramme d'un mode de réalisation préféré de procédé selon l'invention,
- la figure 2 illustre plusieurs courbes de fusion 12 dites « de référence », pour un apprentissage du mode de réalisation de procédé selon l'invention,
- la figure 3 illustre :

  ◦ plusieurs courbes de fusion normalisées 13 (réplicats « techniques ») dites « de mesure » d'un échantillon dont on cherche à déterminer l'appartenance à un groupe déterminé parmi plusieurs groupes possibles,
  ◦ les percentiles 14 de ces courbes de mesure
  ◦ les dérivées premières 15 de ces courbes de mesure
  ° les dérivées secondes 16 de ces courbes de mesure

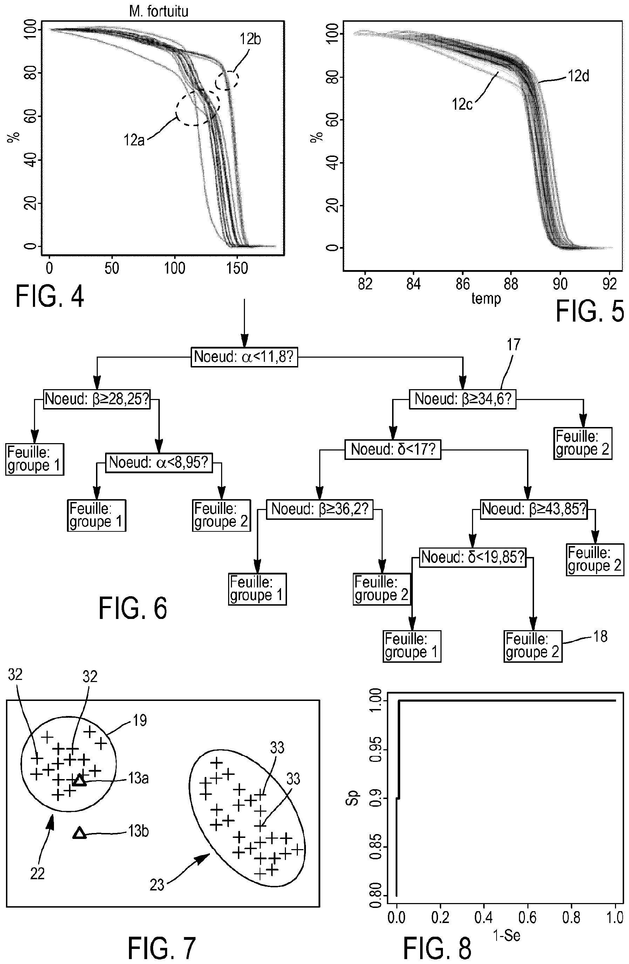

- la figure 4 illustre plusieurs courbes de fusion de référence (réplicats techniques) de plusieurs échantillons (réplicats biologiques) appartenant tous au groupe initial « *Mycobacterium fortuitum* »,
- la figure 5 illustre plusieurs courbes de fusion de référence (réplicats techniques) de plusieurs échantillons (réplicats biologiques) appartenant au groupe initial « *M. avium*» ou « *M. szulgai* »,
- la figure 6 illustre un exemple d'un arbre d'une forêt aléatoire, généré dans un cas à trois descripteurs a, $\beta$ et $\delta$ et avec deux groupes possibles (groupe 1 et groupe 2) ; la réponse affirmative à la question de chaque nœud correspond à choisir la branche subséquente de gauche ; la réponse négative à la question de chaque nœud correspond à choisir la branche subséquente de droite ;
- la figure 7 illustre graphiquement le principe d'un indicateur de confiance selon l'invention,
- la figure 8 est une courbe ROC associée à l'utilisation de l'indice de confiance basé sur les proximités intra-groupe pour discriminer les observations affectées au bon groupe ou non, et

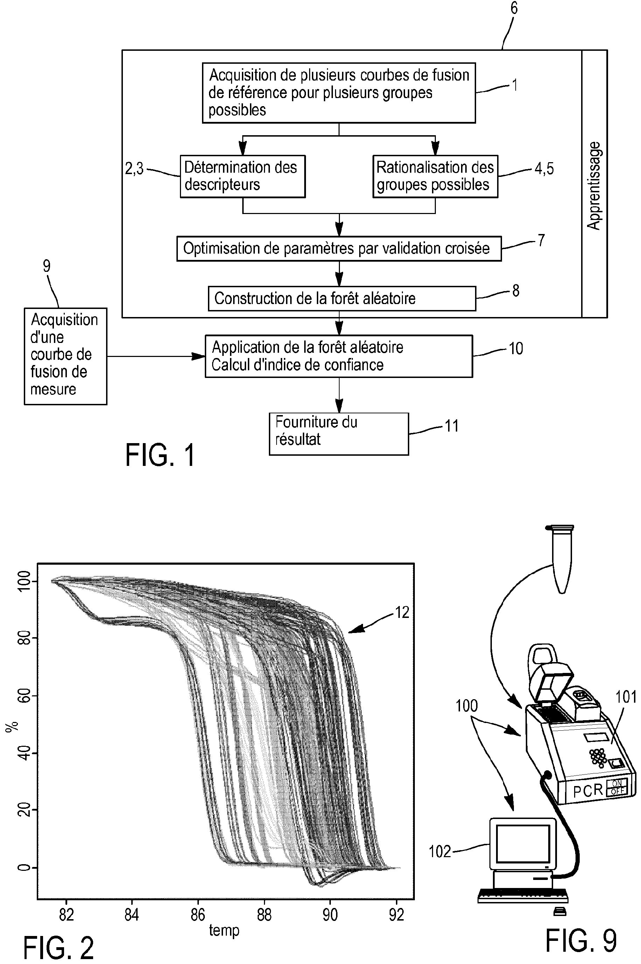

- la figure 9 illustre schématiquement un mode de réalisation de dispositif 100 selon l'invention.

On va donc décrire, en référence aux figures 1 à 8, un mode de réalisation préféré de procédé selon l'invention. Dans la suite de la présente description, l'expression « procédé selon l'invention » désignera uniquement ce mode de réalisation nullement limitatif.

L'objectif de ce mode de réalisation est de pouvoir discriminer différentes espèces. On choisit pour exemple nullement limitatif une discrimination de différentes espèces du genre *Mycobacterium.*

Dans ce mode de réalisation, un "échantillon biologique" correspond à tout type d'échantillon contenant, ou susceptible de contenir, de la matière biologique. De préférence, il s'agit d'un échantillon susceptible de contenir des mycobactéries et/ou d'un échantillon susceptible de contenir de l'acide désoxyribonucléique (ou « ADN »), ou des traces d'ADN de mycobactéries.

**Principe d'obtention d'une courbe de fusion.**

Dans ce mode de réalisation, la technique de biologie moléculaire dite de « fusion d'ADN à haute résolution » est également nommée "HRM" (pour « high resolution melting »). Cette technique HRM est réalisée à partir d'ADN double brin. Avant l'analyse par HRM, un fragment de l'ADN, dans lequel sont susceptibles d'être localisées des mutations d'intérêt, est amplifié par une réaction PCR (pour « polymerase chain réaction »). L'échantillon contient alors un grand nombre de copies du fragment d'ADN ciblé et amplifié par la réaction PCR.

L'analyse HRM consiste ensuite à chauffer de manière précise et contrôlée le fragment d'ADN amplifié par PCR pour provoquer sa dénaturation. Le suivi de la dénaturation de l'ADN, lors de l'analyse HRM, permet ainsi de déterminer un profil de fusion spécifique du fragment d'ADN cible.

Le « profil de fusion » (aussi appelé « courbe de fusion ») correspond à l'évolution de la dénaturation d'une (ou en moyenne de chaque) molécule d'ADN en fonction de la température. Au sens de l'invention, une courbe de fusion n'est pas nécessairement une courbe graphique, mais peut être une liste ou un tableau de valeurs de plusieurs points de cette courbe au cours de cette dénaturation d'une (ou en moyenne de chaque) molécule d'ADN en fonction de la température.

La réaction PCR comprend par exemple la répétition du cycle constitué des 3 étapes suivantes :

- une étape à une température de 90°C à 99°C, pendant 5 à 30 secondes,
- suivie d'une étape à une température de 58°C à 64°C, pendant 5 à 30 secondes,
- suivie d'une étape à une température de 70 à 74°C, pendant 5 secondes à 1 minute.

Les trois étapes constituant le cycle de PCR correspondent respectivement à des étapes :

- de dénaturation de l'ADN (de 90°C à 99°C),
- d'hybridation de l'ADN avec les amorces (de 58°C à 64°C),
- et d'élongation de l'ADN par l'ADN polymérase à partir des amorces (de 70°C à 74°C).

Le cycle est typiquement répété de 40 à 50 fois, de préférence 45 fois.

Ladite réaction PCR est de préférence précédée d'une étape de dénaturation initiale de l'ADN contenu dans ledit échantillon biologique, de préférence à 95°C pendant 10 minutes.

Cette étape de dénaturation initiale est une étape de chauffage effectuée avant le cycle PCR. Elle permet de préparer l'ADN de l'échantillon, qui va servir de matrice durant la réaction d'amplification, notamment en déshybridant complètement l'ADN double brin, en cassant les structures secondaires de l'ADN ou encore en activant l'ADN polymérase.

Ladite réaction PCR est par exemple effectuée en utilisant un mélange réactionnel comprenant au moins :

- l'ADN contenu dans l'échantillon biologique
- du $MgCl_2$ à raison de 3 mM,
- les amorces à raison de 0,4 $\mu$M, et
- au moins une unité d'ADN polymérase.

Ladite réaction PCR est par exemple suivie d'une étape de chauffage progressif entre 60°C et 100°C, de préférence de 65°C à 95°C, pour réaliser une dénaturation dudit produit d'amplification, et obtenir un profil de fusion dudit produit d'amplification.

Cette étape de chauffage progressif correspond à un chauffage de l'échantillon réalisé de manière contrôlée, au cours duquel la température augmente progressivement par paliers au cours du temps, comme par exemple une augmentation de 0,2°C/seconde.

La dénaturation dudit produit d'amplification est typiquement suivie à l'aide d'un marqueur fluorescent, de préférence choisi parmi le LC Green, le LC Green Plus, le ResoLight, l'EvaGreen, le Chromofy, et le SYTO 9.

Les étapes d'amplification et de fusion ont été réalisées en utilisant le kit de fusion haute résolution LightCycler® 480 master kit (Roche). Le mélange réactionnel est composé de 2X Master Mix, $MgCl_2$, d'amorces sens et antisens, d'ADN génomique et d'eau, dans un volume final de 10 $\mu$l. La procédure d'amplification consiste en une dénaturation initiale suivie par 45 cycles de dénaturation, hybridation et élongation. Après amplification, le programme de fusion est réalisé par chauffage à 95°C pendant 1 minute, refroidissement à 40°C pendant 1 min, suivi de l'application d'une augmentation de température de 65 à 95°C avec une vitesse de palier de 0,2°C/s et d'une mesure de la fluorescence en continu. Chaque réaction a été effectuée en triple dans des plaques à 96 puits, avec le système LightCycler® 480 (Roche). Chaque analyse HRM inclut un contrôle négatif où la matrice d'ADN a été remplacée par de l'eau.

On notera que, de manière avantageuse pour l'invention, il est plus facile d'obtenir une courbe de fusion que de mesurer l'expression d'un sous-ensemble de gènes.

### Apprentissage

Comme illustré sur la figure 1, le mode de réalisation de procédé selon l'invention comprend un apprentissage 6 comprenant :

- une acquisition 1 de différentes courbes de fusion d'ADN, dites courbes de référence, à partir de différents échantillons biologiques de référence appartenant à différents « groupes initiaux » connus et déterminés avant l'apprentissage, puis

- une détermination 2, 3 de descripteurs à partir des courbes de référence, puis
- une construction 8 d'une forêt selon la méthode des forêts aléatoires, comprenant une construction de plusieurs arbres selon la méthode des forêts aléatoires, chaque arbre comprenant, à chaque nœud, l'utilisation d'un des descripteurs associé à un seuil et permettant de séparer les courbes de référence en deux sous-ensembles, chaque feuille de chaque arbre ne correspondant qu'à un seul groupe parmi les différents groupes possibles. Dans un arbre de classification une feuille correspond toujours à un seul groupe qui est, en général, le groupe majoritaire des observations qui "tombent" dans cette feuille. La particularité des feuilles pures est que toutes les observations de l'échantillon de référence qui "tombent" dans cette feuille appartiennent au même groupe.

Les « groupes possibles », parmi lesquels on va ensuite chercher à classer un échantillon biologique inconnu, consistent en les différents « groupes initiaux » des différents échantillons biologiques de référence utilisés lors de l'étape d'apprentissage, éventuellement modifiés (par exemple par au moins une étape de séparation de groupe et/ou au moins une étape d'unification de groupes tels que décrits par la suite pour la rationalisation des groupes). De préférence, les « groupes possibles » comprennent au moins une partie des différents « groupes initiaux »

Cette phase d'apprentissage est réalisée une fois pour chaque type d'application (avec possibilité éventuelle de répétition de cette phase pour inclusion de nouveaux échantillons de référence et/ou de nouveaux groupes). Elle a pour objectif de définir les groupes possibles (finaux) et de construire la règle de décision, avec :

- en entrées de l'étape d'apprentissage : librairie de profils de fusion d'apprentissage normalisés avec affectation, pour chacun, à un groupe initial prédéfini. Chaque groupe initial doit être représenté par plusieurs réplicats biologiques. Chaque réplicat biologique doit être représenté par plusieurs réplicats techniques.
- en sorties de l'étape d'apprentissage : règle de décision d'affectation

Importation du signal normalisé : pour l'étape d'acquisition 1 des différentes courbes de fusion d'ADN « de référence » servant à l'apprentissage, on utilise le protocole d'obtention de courbe de fusion précédemment décrit et en appliquant une méthode de normalisation telle que proposée par exemple par le logiciel associé au LightCycler® 480 (Roche), 6 séries d'expériences ont été menées à différentes dates permettant la production de 417 profils HRM (c'est-à-dire 417 courbes de fusion de référence) correspondant à 19 espèces (ou « groupes initiaux ») de *Mycobacterium* différentes. Chaque espèce est représentée par plusieurs réplicats techniques de plusieurs réplicats biologiques (2 à 20 réplicats biologiques par espèce). On appelle « réplicats biologiques » les différents échantillons biologiques provenant de différents individus d'une même espèce. On appelle « réplicats techniques » d'un même réplicat biologique les différentes courbes de fusion obtenues à partir du même échantillon biologique. L'entrée du logiciel est un fichier texte contenant les coordonnées des profils de fusion après normalisation par le logiciel en sortie de machine.

La répartition des réplicats biologiques entre les espèces est donnée dans la Table 1 et la représentation des courbes de référence normalisées 12 associées à l'ensemble des réplicats techniques pour les différents réplicats biologiques est donnée dans la Figure 2.

Table 1

| Espèce | Nombre de réplicats biologiques |
|---|---|
| "M. abscessus" | 3 |
| "M. avium" | 20 |
| "M. bohemicum" | 4 |
| "M. bovis" | 4 |
| "M. chelonae" | 4 |
| "M. flavescens" | 3 |
| "M. fortuitum" | 5 |
| "M. gastri" | 4 |
| "M. gordonae" | 5 |
| "M. haemophilum" | 3 |
| "M. immunogenicum" | 3 |
| "M. kansasii" | 9 |

(suite)

| Espèce | Nombre de réplicats biologiques |
|---|---|
| "M. marinum" | 4 |
| "M. phlei" | 4 |
| "M. phocaicum" | 4 |
| "M. pseudoschottsi" | 3 |
| "M. smegmatis" | 6 |
| "M. szulgai" | 4 |
| "M. wolinski" | 3 |
| TOTAL | 95 |

Ces 19 espèces forment les 19 groupes initiaux.

Détermination des descripteurs : ensuite, on procède à la détermination des descripteurs. La détermination 2, 3 des descripteurs comprend d'abord une détermination préliminaire 2 des descripteurs à partir des courbes de fusion « de référence » D(T) (Dénaturation « D » de l'ADN (typiquement en % ou en signal de fluorescence) en fonction de la température « T »), pour laquelle on considère :

- Les points de mesure de chaque courbe de fusion normalisée (180 points dans le cas présent). On obtient ainsi 180 descripteurs qui permettent de caractériser chaque courbe de fusion.
- les percentiles (101 quantiles, de 0 à 100): on nomme i$^{ème}$ percentile, la température d'une courbe de fusion normalisée à laquelle i% de la dénaturation de l'ADN a eu lieu. On obtient ainsi 101 descripteurs (obtenus par interpolation entre les points de mesure) correspondant aux 101 percentiles, et qui permettent de caractériser chaque courbe de fusion.

On adjoint en outre aux courbes de fusion *stricto sensu,* pour la détermination préliminaire 2 des descripteurs, des données dérivées permettant de décrire les courbes avec plus de précision :

- la dérivée numérique première de chaque courbe de fusion normalisée $\frac{dD(T)}{dT}$. On obtient ainsi, pour une courbe de fusion établie initialement sur 180 points (i.e. 180 valeurs de taux de dénaturation), 178 descripteurs supplémentaires, qui permettent de caractériser chaque dérivée première de courbe de fusion.

- la dérivée numérique seconde de chaque courbe de fusion normalisée $\frac{d^2D(T)}{dT^2}$. On obtient ainsi, pour une courbe de fusion établie initialement sur 180 points (i.e. 180 valeurs de taux de dénaturation), 176 descripteurs supplémentaires, et qui permettent de caractériser chaque dérivée seconde de courbe de fusion.

On obtient finalement :

180+101+178+176= 635 descripteurs pour décrire chaque courbe de fusion ou réplicat technique.

La détermination 2, 3 des descripteurs comprend :

- la détermination préliminaire 2 de descripteurs précédemment décrite, puis
- une élimination 3 de certains descripteurs redondants.

La redondance de l'information est préjudiciable à l'apprentissage des groupes possibles. Or, il existe de très fortes corrélations entre valeurs successives sur une courbe de fusion ou sur ses dérivées. C'est pourquoi n'est conservé qu'un seul descripteur par ensemble de descripteurs exhibant deux à deux un coefficient de corrélation de Pearson supérieur à 0,95. Ainsi, l'élimination 3 de certains descripteurs comprend, pour chaque ensemble de descripteurs exhibant deux à deux un coefficient de corrélation de Pearson supérieur à 0,95, une conservation d'un seul descripteur. On retient finalement 208 descripteurs (parmi les 635 initiaux) après élimination des descripteurs redondants, dont :

- 38 points de courbes de fusion
- 12 percentiles
- 59 points de la dérivée première
- 99 points de la dérivée seconde.

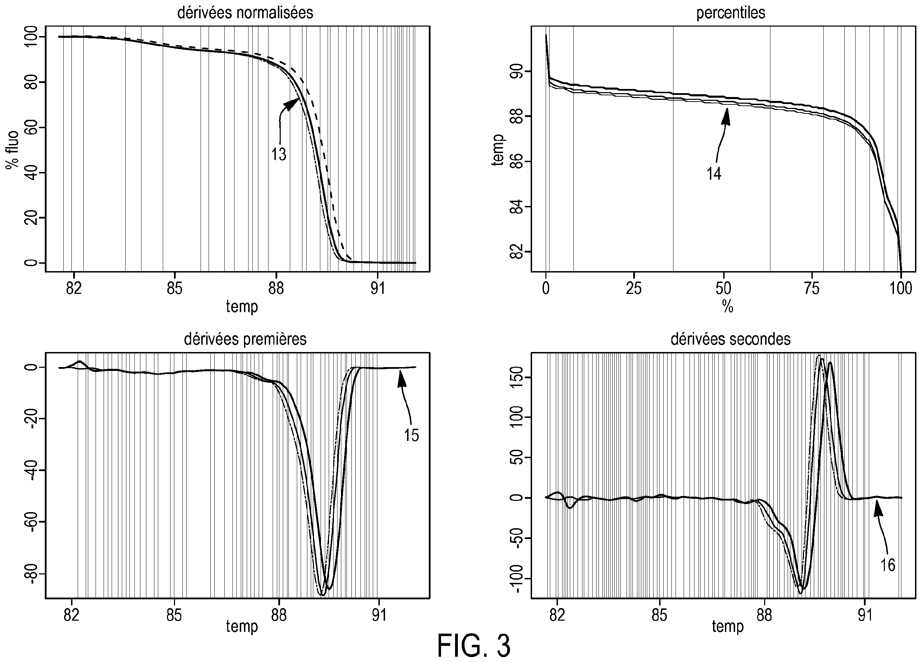

L'emplacement de chaque descripteur sélectionné est donné par des traits verticaux sur la Figure 3.

Ceci illustre bien l'avantage du procédé selon l'invention : on voit en effet que les dérivées (première et seconde, en particulier seconde) des courbes de fusion sont très riches en informations discriminantes permettant de déterminer qu'un échantillon biologique appartient à un groupe possible donné, car elles comprennent une grande partie des descripteurs finalement retenus. Cela se traduit par une discrimination plus fine des profils de fusion.

Rationalisation des groupes :

Le mode de réalisation de procédé selon l'invention peut être appliqué à un grand nombre de problèmes ou d'applications de complexité d'apprentissage variée. Il peut être amené à devoir discriminer des groupes génétiquement plus ou moins proches. Il est donc impossible, a priori, de savoir si tous les groupes initiaux seront différentiables par leurs courbes de fusion. C'est pourquoi, lors de l'apprentissage une étape de « rationalisation des groupes » est insérée. Elle permet de définir le périmètre des groupes initiaux différenciables ou non. Cette étape est le résultat de deux constatations principales :

- certains groupes initiaux sont constitués de sous-groupes hétérogènes ;
- certains groupes initiaux ne sont pas différentiables les uns des autres par leurs courbes de fusion.

Sur la Figure 2, on constate que :

1) les réplicats biologiques ou techniques peuvent présenter des profils très différents à l'intérieur d'un même groupe initial. Ce phénomène apparaît dans deux des groupes initiaux, notamment le groupe initial « M. fortuitum » illustré la Figure 4. Ainsi chacun de ces deux groupes initiaux est donc séparé en plusieurs (deux dans le cas présent) sous-groupes possibles pour l'apprentissage, cela permet de faciliter le travail d'apprentissage ; et

2) tous les réplicats biologiques ou techniques de groupes initiaux différents peuvent être assez compacts.

Ainsi, dans le cas 1) ci-dessus, le mode de réalisation de procédé selon l'invention (plus précisément l'apprentissage 6) comprend :

- après l'acquisition de différentes courbes de référence (mais de préférence avant la construction des arbres), une identification de plusieurs courbes de référence correspondant au même groupe initial, dit groupe ambigu (comme par exemple le groupe initial « M. fortuitum » (figure 4), le groupe initial « M. kansasii »), et présentant des profils séparés en plusieurs sous-groupes ; cette identification est par exemple mise en œuvre en utilisant un critère de distance intra-groupe pour lequel est défini un seuil, et
- une séparation 4 de ce groupe ambigu en plusieurs groupes possibles 12a et 12 b.

De même, dans le cas 2) ci-dessus, le mode de réalisation de procédé selon l'invention (plus précisément l'apprentissage 6) comprend :

- après l'acquisition de différentes courbes de référence (mais de préférence après la construction des arbres, la fusion ou unification des groupes pouvant nécessiter une première construction des arbres), une identification de plusieurs courbes de référence correspondant à plusieurs groupes initiaux, dit groupes confondus, et présentant des profils réunis en un seul groupe ; cette identification est par exemple mise en œuvre en utilisant des taux d'erreur d'apprentissage : si on fait des erreurs sur un groupe, on peut associer des observations mal classées aux observations dont elles sont le plus proche pour former un groupe fusionné ou unifié, et
- une unification 5 de ces groupes confondus en un unique groupe possible ;

Dans ce mode de réalisation de procédé selon l'invention, les groupes initiaux « M. szulgai » 12c et « M. avium » 12d et sont par exemple très proches, mais ne sont au final pas unifiés malgré leur proximité, grâce à la grande finesse d'analyse du procédé selon l'invention.

On obtient au final les 21 groupes possibles finaux suivants listés dans la table 2:

Table 2

| Nom de chacun des groupes possibles finalement utilisés dans la méthode des forêts aléatoires |
|---|
| "M. abscessus" |
| "M. avium" |
| "M. bohemicum" |
| "M. bovis" |
| "M. chelonae" |
| "M. flavescens" |
| "M. fortuitum 1" |
| "M. fortuitum 2" |
| "M. gastri" |
| "M. gordonae" |
| "M. haemophilum" |
| "M. immunogenicum" |
| "M. kansasii 1" |
| "M. kansasii 2" |
| "M. marinum" |
| "M. phlei" |
| "M. phocaicum" |
| "M. pseudoschottsi" |
| "M. smegmatis" |
| "M. szulgai" |
| "M. wolinski" |

L'étape de « rationalisation des groupes » peut, en outre, être itérative, après construction de la forêt aléatoire décrite ci-après. Dans un premier temps, après optimisation des paramètres, on applique la méthode des Forêts Aléatoires adaptée en validation croisée en deux blocs. On repère alors les réplicats biologiques qui sont affectés au mauvais groupe. Pour chacun de ces réplicats, on crée un nouveau groupe rassemblant ce réplicat mal affecté et le réplicat biologique le plus proche du groupe affecté à tort. On a donc créé un groupe « hybride » comportant une double étiquette. On recommence jusqu'à ce que tous les réplicats biologiques de l'échantillon d'apprentissage soient correctement affectés. On obtient à l'issue de cette étape un certain nombre de groupes ayant une ou plusieurs « étiquettes ».

Bien entendu, cette étape peut comprendre la création de groupes hybrides comportant plusieurs groupes initiaux. Mais, il est très précieux dans un contexte de prédiction avec un grand nombre de groupes d'avoir la possibilité de réduire de façon importante le nombre de possibilités. D'autant plus qu'avec cette méthode, on ne force pas l'ensemble du groupe à fusionner avec un autre mais on raisonne à l'échelle du réplicat biologique. Ainsi, si un groupe initial est hétérogène avec un sous-ensemble de réplicats biologiques qui se rapproche d'un autre groupe, on obtiendra au final deux groupes possibles finaux: un groupe final ne comportant que des réplicats du groupe initial et un groupe final hybride.

Définition d'une méthode de prédiction et détermination des paramètres de la méthode d'apprentissage :

L'apprentissage 6 comprend enfin la construction 8 de la forêt selon la méthode des forêts aléatoires.

Le fonctionnement de cette méthode des forêts aléatoires est ici adapté à la structure des données en réplicats techniques / réplicats biologiques selon l'invention. Les réplicats techniques permettent de rendre compte de la variabilité technique de l'obtention des profils de fusion (variabilité assez limitée). La variabilité biologique est au cœur de l'apprentissage car elle reflète la variabilité à laquelle le mode de réalisation de procédé selon l'invention sera confronté en conditions réelles d'utilisation. Elle est liée aux différences de séquences que l'on peut observer entre individus d'un même groupe possible.

Pour discriminer les k différents groupes possibles (k=21 groupes possibles dans cet exemple, cf table 2), on utilise donc la méthode bien connue des forêts aléatoires (cf références [2], [3], [4] pour plus de détails concernant les généralités bien connues de cette méthode des forêts aléatoires). Cette méthode, basée sur les arbres de classification, a pour principe de construire plusieurs arbres de classification en utilisant pour chaque arbre un sous-ensemble des n courbes de fusion de référence (aussi appelées « observations ») de départ (n=417 courbes de fusion de référence dans cet exemple) et, pour chaque nœud de l'arbre, un sous-ensemble des p descripteurs (aussi appelées « variables ») de départ (p=208 descripteurs dans cet exemple). Cette méthode dépend de deux paramètres :

- *ntree* le nombre d'arbres construits, c'est-à-dire le nombre total d'arbres impliqués dans la règle de décision finale et
- *mtry* le nombre de descripteurs étudiés à chaque nœud de chaque arbre, avec *mtry* ≤ *p,* c'est-à-dire le nombre de descripteurs aléatoirement choisis parmi l'ensemble des descripteurs disponibles lors de l'apprentissage de chaque nœud de chaque arbre.

Ces deux paramètres sont déterminés lors d'une étape d'optimisation 7 par validation croisée en deux blocs sur les données d'apprentissage (courbes de référence). Pour cette étape 7 (qui fait partie de l'apprentissage 6), et pour chaque utilisation de validation croisée, on travaille à l'échelle du réplicat biologique, c'est-à-dire qu'à chaque étape de la validation croisée, les réplicats techniques d'un réplicat biologique sont soit tous affectés au bloc d'apprentissage, soit tous affectés au bloc de validation. Cette contrainte a l'avantage de mimer au plus près les conditions réelles d'apprentissage. Les paramètres choisis sont ceux qui maximisent les pourcentages de bien classés moyens obtenus sur 100 répartitions aléatoires en blocs apprentissage/test. Ainsi, pour chaque valeur possible du couple (*ntree, mtry*), on construit une forêt (avec plusieurs arbres) selon la méthode des forêts aléatoires sur la base de la moitié des n observations, puis on teste cette forêt sur l'autre moitié des n observations dont on connait déjà en réalité l'appartenance ou non à chacun des k groupes possibles ; on sélectionne alors la valeur du couple (*ntree, mtry*) ayant construit les forêts qui donnent en moyenne (puisqu'on réalise 100 répartitions aléatoires dont 100 forêts pour chaque couple de valeurs) les meilleurs résultats. On obtient un nombre optimal de *ntree*=1000 arbres et de *mtry*=10 variables par nœud.

On construit alors les *ntree*=1000 arbres de la forêt aléatoire en utilisant les n=417 observations.

Pour la construction de chaque arbre :

- on tire aléatoirement 100 observations (ou courbes de référence) parmi les n=417 disponibles, et
- à chaque nœud 17 de chaque arbre, on tire aléatoirement *mtry*= 10 descripteurs parmi les p=208 disponibles, et on sélectionne, parmi les *mtry* = 10 descripteurs tirés, le descripteur et le seuil associé qui ensemble optimisent l'indice de Gini; il y a autant de nœuds que nécessaire pour que chaque fin de ramification 18 ou « feuille » 18 soit pure, c'est-à-dire que les observations qui y "tombent" appartiennent toutes au même groupe initial.

La figure 6 illustre un exemple d'arbre d'une forêt aléatoire, dans un cas à trois descripteurs α, β et δ (p=3) et avec deux groupes possibles (k=2, groupe 1 et groupe 2). Cet arbre, même s'il ne correspond pas au cas du mode de réalisation de procédé selon l'invention de la figure 2 (k=21 groupes possibles, p=208 descripteurs, avec des arbres beaucoup trop grands pour être illustrés) illustre parfaitement le principe des arbres construits dans ce mode de réalisation de procédé selon l'invention.

Chaque nœud 17 correspond à une question posée par rapport à un descripteur, typiquement : est ce que ce descripteur a une valeur inférieure (ou inférieure ou égale) à un seuil ?

Par exemple :

- Pour chacun des descripteurs faisant partie des points de courbes de fusion (38 descripteurs) : est-ce que le pourcentage de dénaturation (ou autre unité arbitraire de la courbe de fusion), pour une valeur de température spécifique à ce descripteur, a une valeur inférieure (ou inférieure ou égale) à un seuil donné ?
- Pour chacun des descripteurs faisant partie des percentiles (12 descripteurs) : est-ce que la température, pour une valeur de pourcentage de dénaturation (ou autre unité arbitraire de la courbe de fusion) spécifique à ce descripteur, a une valeur inférieure (ou inférieure ou égale) à un seuil donné?
- Pour chacun des descripteurs faisant partie des points de la dérivée première (59 descripteurs) : est-ce que la dérivée première de la courbe de fusion, pour une valeur de température spécifique à ce descripteur, a une valeur inférieure (ou inférieure ou égale) à un seuil donné?
- Pour chacun des descripteurs faisant partie des points de la dérivée seconde (99 descripteurs) : est-ce que la dérivée seconde de la courbe de fusion, pour une valeur de température spécifique à ce descripteur, a une valeur inférieure (ou inférieure ou égale) à un seuil donné?

Chaque feuille 18 correspond à un des k groupes possibles finaux.

Apprentissage de l'indice de confiance :

Par construction, la méthode des Forêts Aléatoires permet de calculer des proximités entre observations par l'étude du nombre d'arbres dans lesquels deux observations « tombent » dans la même feuille. Cette proximité est utilisée pour calculer un indicateur de confiance de la prédiction et donc pour refuser éventuellement d'affecter une observation à un des groupes possibles.

Ainsi, après la construction de la forêt aléatoire, lors de la phase d'apprentissage 6, on calcule la distribution des proximités deux à deux de tous les couples de réplicats biologiques de la librairie d'apprentissage appartenant à un même groupe possible. On définit les proximités entre réplicats biologiques par la valeur minimale des proximités calculées entre ses réplicats techniques (méthode dite du lien complet). Cette distribution peut être lissée par une méthode à noyaux. On répète cette opération pour chaque groupe possible, on obtient ainsi une distribution des distances intra-groupes spécifique à chaque groupe.

**Prédiction : classification d'un échantillon biologique inconnu, dit « de mesure »**

La prédiction est l'étape courante du mode de réalisation de procédé selon l'invention. Elle a pour objectif d'appliquer la règle de décision à un échantillon biologique afin d'obtenir une affectation à l'un des groupes possibles finaux (aussi appelés « classes ») obtenus en sortie de l'apprentissage (notamment après l'étape de rationalisation des groupes), cette affectation étant assortie d'un indicateur de confiance. On a donc :

- en entrées de l'étape de prédiction : un ensemble de profils de fusion (réplicats techniques) obtenus à partir d'un même échantillon biologique « de mesure ».
- en sorties de l'étape de prédiction : décision d'affectation de l'échantillon « de mesure » avec indicateur de confiance, ou éventuellement un refus d'affectation à l'un quelconque des groupes possibles.

En effet, l'objectif du mode de réalisation de procédé selon l'invention est ensuite, à partir de la description d'échantillons « de mesure » par leur courbe de fusion, de décider d'affecter ou non cet individu à l'un des k groupes possibles finaux définis lors de l'apprentissage (méthode supervisée) et d'affecter un indicateur de confiance à la décision proposée.

Ainsi, le mode de réalisation de procédé selon l'invention comprend une acquisition (9) d'au moins une courbe normalisée (comme vu précédemment) de fusion d'ADN de l'échantillon biologique de mesure, dite au moins une courbe de mesure, chaque courbe de mesure comprenant différents points, chaque point correspondant à une grandeur proportionnelle (par exemple un signal de fluorescence) ou représentative d'un taux (typiquement en %) ou d'une quantité de dénaturation de l'ADN de l'échantillon de mesure en fonction d'une température ; cette acquisition peut comprendre la réalisation de la PCR et de la courbe de fusion elle-même (en laboratoire), et/ou un simple téléchargement de données (informatiques par exemple) de cette courbe de fusion.

De manière optionnelle, la réalisation de la PCR pour cette courbe de fusion peut être obtenue en présence simultanée de plusieurs couples d'amorces ciblant plusieurs molécules cibles d'ADN. On parle alors de conditions de « multiplexage ».

Le mode de réalisation de procédé selon l'invention comprend en outre une détermination 10, par la méthode des forêts aléatoires basée sur la forêt d'arbres construite lors de la phase d'apprentissage, d'une appartenance de l'échantillon biologique de mesure à un groupe déterminé parmi k différents groupes possibles finaux. Cette détermination comprenant une analyse, par la méthode des forêts aléatoires basée sur la forêt d'arbres construite lors de la phase d'apprentissage, de descripteurs issus de l'au moins une courbe de mesure, les descripteurs comprenant :

◦ un ou plusieurs points de chaque courbe de mesure et/ou
◦ tout ou partie des 101 percentiles des taux de dénaturation de chaque courbe de mesure, et/ou
◦ un ou plusieurs points (typiquement au moins 30 points) de la dérivée première de chaque courbe de mesure et/ou
◦ un ou plusieurs points (typiquement au moins 30 points) de la dérivée seconde de chaque courbe de mesure.

Les réplicats techniques de l'échantillon biologique de mesure sont soumis indépendamment à la forêt aléatoire et un groupe possible est affecté à chacun d'eux. Par défaut, l'échantillon biologique de mesure est affecté au groupe majoritaire parmi les groupes prédits pour chaque réplicat technique. En cas de groupes multiples, l'indice de confiance pourra être utilisé pour trancher.

Les forêts aléatoires étant des méthodes stochastiques (plusieurs applications peuvent donner des résultats différents), on applique plusieurs fois (3 fois dans ce mode de réalisation) cette méthode pour prédire l'affectation de l'échantillon biologique.

L'emplacement de chaque descripteur est donné par des traits verticaux sur la Figure 3.

La figure 3 illustre :

- en haut à gauche, trois courbes de fusion de mesure 13 (trois réplicats techniques) d'un même échantillon de mesure dont on cherche à déterminer l'appartenance à l'un des k groupes possibles;
- en haut à droite, trois courbes 14 illustrant les percentiles correspondants aux trois réplicats techniques 13;
- en bas à gauche, les trois dérivées premières 15 des trois courbes de fusion de mesure 13, et
- en bas à droite, les trois dérivées secondes 16 des trois courbes de fusion de mesure 13.

Calcul d'un indicateur de confiance :

La qualité du mode de réalisation de procédé selon l'invention est conditionnée par la qualité de la librairie initiale d'apprentissage. Plus celle-ci est riche de variabilité biologique, plus l'apprentissage sera précis et généralisable à une grande diversité de nouveaux échantillons.

Toutefois, quelle que soit la qualité de la librairie d'apprentissage, lors de la prédiction de nouveaux échantillons, il est toujours possible de rencontrer des échantillons qui lui sont totalement étrangers. Dans ce cas-là, les méthodes classiques d'apprentissage fourniront tout de même une prédiction en affectant le nouvel échantillon au groupe possible dont il est le plus proche. Le mode de réalisation de procédé selon l'invention doit être capable de refuser d'affecter un nouvel échantillon à quelque groupe possible que ce soit.

Pour cela, le mode de réalisation de procédé selon l'invention comprend un calcul d'un indice de confiance de l'étape de détermination d'une appartenance de l'échantillon biologique de mesure à un groupe déterminé.

L'objectif de cette étape de calcul d'un indicateur de confiance est double :

- quantifier la fiabilité des prédictions rendues ;
- autoriser un réplicat biologique à prédire à n'être affecté à aucun groupe possible.

Les forêts aléatoires ont l'avantage de fournir des mesures de proximité entre observations. Pour plus de détails sur cette notion bien connue de « proximité » dans la méthode des forêts aléatoires, on peut par exemple se référer aux références [3] et [4].

Ces mesures sont utilisées pour fournir l'indice de confiance. En effet, si l'observation à prédire est proche des observations du groupe possible auquel elle est affectée alors la qualité de la classification est potentiellement meilleure que si l'observation à prédire est éloignée des observations du groupe possible auquel elle est affectée. Ce principe a été utilisé pour construire l'indice de confiance.

Sur les données d'apprentissage, on a calculé la distribution des proximités moyennes des courbes de fusion de référence d'un même groupe possible. Ensuite, quand un échantillon biologique de mesure est affecté à un groupe possible, on calcule sa proximité moyenne aux réplicats biologiques de ce groupe et on la compare aux proximités des courbes de fusion de référence de ce groupe possible. On peut alors calculer le pourcentage de courbes de fusion de référence dont la proximité est inférieure à celle de la courbe de fusion de mesure à prédire. Ce pourcentage est une estimation de la probabilité d'appartenance au groupe prédit et est utilisé comme indice de confiance.

La figure 7 illustre le principe de cet indice avec deux groupes possibles 22, 23 auxquels appartiennent des courbes de fusion de référence (croix) respectivement 32 et 33.

Dans le cas où la (ou chacune des ou la moyenne des) courbe(s) de mesure 13a (triangle) :

- est plus proche du groupe 22 que du groupe 23 et
- a sa proximité aux courbes de fusion 32 du groupe 22 conforme aux proximités que l'on observe entre les courbe de fusion 32 du groupe 22 ;

alors l'échantillon correspondant aux courbes 13a est considéré comme appartenant au groupe 22, et le mode de réalisation de procédé selon l'invention confirme que le groupe déterminé est bien le groupe 22.

Dans le cas où la (ou chacune des ou la moyenne des) courbe(s) de mesure 13b (triangle) :

- est plus proche du groupe 22 que du groupe 23, mais
- a sa proximité aux courbes de fusion 32 du groupe 22 bien inférieure aux proximités que l'on observe entre courbes de fusion 32 du groupe 22,

alors l'échantillon correspondant au courbes 13b n'appartient ni au groupe 22 ni au groupe 23, et de préférence le mode de réalisation de procédé selon l'invention comprend un refus d'affecter l'échantillon biologique de mesure au groupe déterminé 22 et même éventuellement à quelque groupe que ce soit.

Lors de la phase d'apprentissage, la distribution des proximités deux à deux de tous les couples de réplicats biologiques de la librairie d'apprentissage appartenant à un même groupe a été calculée.

Lors de l'étape de prédiction, pour toute nouvelle observation (i.e. pour toute nouvelle courbe de fusion « de mesure »), on calcule, par la même méthode, sa proximité moyenne aux réplicats biologiques de référence du groupe auquel il a été affecté. On utilise alors la distribution globale obtenue précédemment pour calculer l'estimation de la probabilité d'appartenance à ce groupe..

Si la nouvelle observation passe cette étape, la probabilité d'appartenance à ce groupe possible est fournie à l'utilisateur en même temps que le groupe prédit.

La possibilité d'appliquer cette dernière étape est bien entendu conditionnée par une taille suffisante de la librairie d'apprentissage.

La Figure 8 montre la courbe ROC (pour « Receiver Operating Characteristic » ou « caractéristique de fonctionnement du récepteur ») associée à cet indice (AUC=0.9988, AUC étant l'aire sous la courbe ROC ou « area under the curve »).

Cette figure 8 est d'excellente qualité. En effet, en référence à la figure 7, si on utilise une valeur seuil égale à 0,14 pour l'indice de confiance alors, toutes les observations ayant un indice inférieur à ce seuil (symbolisée par la boucle 19) sont des observations mal classées alors que seule une observation bien classée à un indice supérieur à ce seuil. Grâce à cet indice on peut donc identifier de façon très satisfaisante les observations bien classées.

Dans le cas d'affectations à plusieurs groupes possibles (suite aux différentes applications des forêts aléatoires ou aux résultats contradictoires des différents réplicats techniques) ou d'un indice de confiance faible, on peut à nouveau calculer la proximité à l'ensemble des groupes possibles prédits au moins une fois (sur l'ensemble des arbres de la forêt) pour un réplicat biologique. Si l'un de ces groupes possibles exhibe un indice supérieur au seuil de 0,14, on peut marquer cette courbe de mesure comme appartenant probablement au groupe possible ayant la valeur d'indice maximale.

En appliquant cette règle aux courbes de référence pour tester son efficacité, on « récupère » des courbes de fusion que l'on avait affecté en majorité à la mauvaise espèce mais pour lesquelles la bonne espèce avait été prédite au moins une fois et avec laquelle l'indice de confiance calculé est supérieur à 0,14.

Enfin, on peut utiliser l'indice de confiance pour départager deux groupes possibles qui auraient été affectés le même nombre de fois à une courbe de fusion de mesure.

Ainsi, pour résumer, le calcul de l'indice de confiance comprend :

- un calcul d'une distribution de proximités moyennes entre courbes de référence appartenant au groupe déterminé,
- un calcul d'une proximité moyenne de l'échantillon biologique de mesure avec les courbes de référence appartenant au groupe déterminé, et
- un calcul d'un taux de courbes de référence appartenant au groupe déterminé, et ayant une proximité moyenne aux autres courbes de référence appartenant au groupe déterminé inférieure à la proximité moyenne de l'au moins une courbe de mesure avec les courbes de référence appartenant au groupe déterminé.

Après l'étape de détermination d'une appartenance de l'échantillon biologique de mesure à un groupe déterminé, le mode de réalisation de procédé selon l'invention comprend (en tant qu'étape de fourniture de résultat 11, typiquement affiché sur un écran ou mémorisé dans une mémoire informatique ou électronique) un refus ou non d'affecter l'échantillon biologique de mesure à quelque groupe possible que ce soit en fonction de la valeur de l'indice de confiance, plus exactement :

- un refus d'affecter l'échantillon biologique de mesure à quelque groupe possible que ce soit si l'indice de confiance est inférieur à une valeur seuil, ou
- une acceptation d'affecter l'échantillon biologique de mesure à un des groupes possibles finaux si l'indice de confiance est supérieur à une valeur seuil

En cumulant les résultats bruts de la forêt aléatoire et l'utilisation de l'indice de confiance, on parvient à bien affecter 95,74% des observations. Quant aux 4,26% d'observations restantes, elles sont clairement identifiées par le mode de réalisation de procédé selon l'invention comme étant suspectes pour ce qui est de leur affectation.

Dans le cas des courbes de la figure 3, l'échantillon est identifié comme appartenant au groupe "abscessus".

Dans ce mode de réalisation de procédé selon l'invention, chacune des étapes suivantes:

- acquisition 1
- détermination des descripteurs 2,3
- rationalisation des groupes 4, 5
- apprentissage 6
- optimisation des paramètres 7
- construction de la forêt aléatoire 8

- acquisition 9
- étape 10 d'analyse des descripteurs, application de la forêt aléatoire, détermination du groupe déterminé parmi les plusieurs groupes possibles, calcul de l'indice de confiance et décision de confirmer l'affectation de l'échantillon biologique de mesure au groupe déterminé ou refus d'affecter l'échantillon biologique de mesure à quelque groupe que ce soit,
- fourniture ou affichage du résultat 11

n'est pas réalisée de manière purement abstraite ou purement intellectuelle mais implique l'utilisation d'un moyen technique (tout comme, de manière générale, toutes les autres étapes de ce mode de réalisation de procédé selon l'invention).

La figure 9 illustre schématiquement un mode de réalisation de dispositif 100 selon l'invention agencé et programmé pour mettre en œuvre ce mode de réalisation de procédé selon l'invention.

Le dispositif 100 comprend des moyens 102 agencés pour et programmés pour mettre en œuvre chacune des étapes suivantes:

- détermination des descripteurs 2,3
- rationalisation des groupes 4, 5
- optimisation des paramètres 7
- construction de la forêt aléatoire 8
- étape 10 d'analyse des descripteurs, application de la forêt aléatoire, détermination du groupe déterminé parmi les plusieurs groupes possibles, calcul de l'indice de confiance et décision de confirmer l'affectation de l'échantillon biologique de mesure au groupe déterminé ou refus d'affecter l'échantillon biologique de mesure à quelque groupe que ce soit
- fourniture ou affichage du résultat 11.

Le dispositif 100 comprend des moyens 101 et 102 agencés pour et/ou programmés pour mettre en œuvre :

- l'étape d'apprentissage 6 et plus précisément l'étape d'acquisition 1 des courbes de référence,
- l'étape d'acquisition 9 d'au moins une courbe de mesure.

Les moyens 102 comprennent un ordinateur, et/ou une unité centrale ou de calcul, et/ou un circuit électronique analogique (de préférence dédié), et/ou un circuit électronique numérique (de préférence dédié), et/ou un microprocesseur (de préférence dédié), et/ou des moyens logiciels. Ces moyens 102 comprennent en outre de préférence un écran ou des moyens d'impression ou des moyens d'export de données pour l'étape 11 de fourniture ou affichage du résultat.

Les moyens 101 comprennent une machine PCR, et/ou selon la variante peut comprendre des moyens informatiques (logiciel combiné à un port USB, un lecteur de carte SD, une connexion à un réseau informatique, etc...) agencés et programmés pour charger et lire des courbes de fusion d'ADN. Ainsi ces moyens 101 sont connectés ou font partie des moyens 102.

Bien sûr, l'invention n'est pas limitée aux exemples qui viennent d'être décrits et de nombreux aménagements peuvent être apportés à ces exemples sans sortir du cadre de l'invention telle que définie par les revendications.

Par exemple, une autre preuve du concept a été réalisée sur d'autres microorganismes incluant *Coxiella burnetii, Chlamydophila spp, Neospora caninum, Toxoplasma gondii,* et *Anaplasmose* avec le même succès. Les expériences ont démontré que la méthode développée permettait d'identifier les différents pathogènes, y compris en conditions de multiplexage c'est-à-dire via une amplification par PCR en présence simultanée de tous les couples d'amorces ciblant les molécules cibles d'ADN de tous les (cinq) pathogènes précédemment cités.

De manière générale, l'invention est applicable sur n'importe quel échantillon biologique, notamment humain, animal, végétal, viral, bactérien, d'archée, fongique, de levure, de viroïde, d'un eucaryote, ou d'un protozoaire.

Références citées :

[1] : WO2013/166373

[2] : « Random Forests », Leo Breiman, paru dans « Machine learning », 45, 5-32, 2001.

[3] : « Classification and Régression by RandomForest », Andy Liaw and Matthew Wiener, R News, vol. 2/3, December 2002, ISSN 1609-3631.

[4] : « Manual on setting up, using, and understanding random Forests V3.1 », Leo Breiman, 2002

**Revendications**

1. Procédé de classification d'un échantillon biologique de mesure, comprenant :

   - une acquisition (9) d'au moins une courbe de fusion d'ADN de l'échantillon biologique de mesure, dite au moins une courbe de mesure,
   - une détermination (10) d'une appartenance de l'échantillon biologique de mesure à un groupe déterminé parmi différents groupes possibles, par une analyse de descripteurs issus de l'au moins une courbe de mesure,

   chacune des étapes d'acquisition et de détermination impliquant l'utilisation d'un moyen technique (101, 102), **caractérisé en ce que** les descripteurs comprennent un ou plusieurs points de la dérivée seconde de chaque courbe de mesure, ledit ou chacun desdits points comprenant une valeur de la dérivée seconde de la courbe de fusion pour une valeur de température spécifique.

2. Procédé selon la revendication 1, **caractérisé en ce que** les descripteurs comprennent au moins 30 points de la dérivée seconde de chaque courbe de mesure.

3. Procédé selon la revendication 1 ou 2, **caractérisé en ce que** les descripteurs comprennent en outre un ou plusieurs points de la dérivée première de chaque courbe de mesure et/ou un ou plusieurs points de chaque courbe de mesure.

4. Procédé selon l'une quelconque des revendications précédentes, **caractérisé en ce que** les descripteurs comprennent en outre un ou plusieurs percentiles de chaque courbe de mesure.

5. Procédé selon l'une quelconque des revendications précédentes, **caractérisé en ce que** la détermination (10) comprend une détermination par une méthode des forêts aléatoires.

6. Procédé selon la revendication 5, **caractérisé en ce qu'**il comprend un apprentissage (6) comprenant :

   - une acquisition (1) de différentes courbes de fusion d'ADN, dites courbes de référence, à partir de différents échantillons biologiques de référence appartenant à différents groupes initiaux, puis
   - une détermination (2, 3) des descripteurs à partir des courbes de référence, puis
   - une construction (8) d'une forêt selon la méthode des forêts aléatoires, comprenant une construction de plusieurs arbres selon la méthode des forêts aléatoires, la ou les variable(s) étudiée(s) à chaque nœud de chaque arbre comprenant un ou plusieurs des descripteurs, chaque feuille de chaque arbre ne correspondant qu'à un seul groupe parmi les différents groupes possibles.

7. Procédé selon la revendication 6, **caractérisé en ce que** la détermination (2, 3) des descripteurs comprend :

   - une détermination préliminaire (2) de descripteurs, puis
   - une élimination (3) de certains descripteurs redondants.

8. Procédé selon la revendication 7, **caractérisé en ce que** l'élimination (3) de certains descripteurs comprend, pour chaque ensemble de descripteurs exhibant deux à deux un coefficient de corrélation de Pearson supérieur à 0,95, une conservation d'un seul descripteur.

9. Procédé selon l'une quelconque des revendications précédentes, **caractérisé en ce qu'**il comprend :

   - après l'acquisition de différentes courbes de référence, une identification de plusieurs courbes de référence correspondant au même groupe initial, dit groupe ambigu, et présentant des profils séparés en plusieurs sous-groupes, et
   - une séparation (4) de ce groupe ambigu en plusieurs groupes possibles.

10. Procédé selon l'une quelconque des revendications précédentes, **caractérisé en ce qu'**il comprend :

    - après l'acquisition de différentes courbes de référence, une identification de plusieurs courbes de référence correspondant à plusieurs groupes initiaux, dit groupes confondus, et présentant des profils réunis en un seul groupe, et
    - une unification (5) de ces groupes confondus en un unique groupe possible.

**11.** Procédé selon l'une quelconque des revendications précédentes, **caractérisé en ce qu'**il comprend en outre un calcul d'un indice de confiance de l'étape de détermination d'une appartenance de l'échantillon biologique de mesure à un groupe déterminé.

**12.** Procédé selon la revendication 11, **caractérisé en ce que** le calcul de l'indice de confiance comprend :

- un calcul d'une distribution de proximités moyennes entre courbes de référence appartenant au groupe déterminé,
- un calcul d'une proximité moyenne de l'au moins une courbe de mesure avec les courbes de référence appartenant au groupe déterminé, et
- un calcul d'un taux de courbes de référence appartenant au groupe déterminé, et ayant une proximité moyenne aux autres courbes de référence appartenant au groupe déterminé inférieure à la proximité moyenne de l'au moins une courbe de mesure avec les courbes de référence appartenant au groupe déterminé.

**13.** Procédé selon la revendication 11 ou 12, **caractérisé en ce qu'**il comprend en outre, après l'étape de détermination d'une appartenance de l'échantillon biologique de mesure à un groupe déterminé, un refus d'affecter l'échantillon biologique de mesure à quelque groupe que ce soit en fonction de la valeur de l'indice de confiance.

**14.** Procédé selon l'une quelconque des revendications précédentes, **caractérisé en ce que** l'acquisition d'au moins une courbe de fusion d'ADN de l'échantillon biologique de mesure comprend l'acquisition d'au moins une courbe de fusion d'un résultat d'une PCR obtenue en présence simultanée de plusieurs couples d'amorces ciblant plusieurs molécules cibles d'ADN.

**15.** Dispositif (100) de classification d'un échantillon biologique de mesure, comprenant :

- des moyens (101) agencés et/ou programmés pour une acquisition (9) d'au moins une courbe de fusion d'ADN de l'échantillon biologique de mesure, dite au moins une courbe de mesure,
- des moyens (102) programmés pour une détermination (10) d'une appartenance de l'échantillon biologique de mesure à un groupe déterminé parmi différents groupes possibles, par une analyse de descripteurs issus de l'au moins une courbe de mesure,

**caractérisé en ce que** les descripteurs comprennent un ou plusieurs points de la dérivée seconde de chaque courbe de mesure, ledit ou chacun desdits points comprenant une valeur de la dérivée seconde de la courbe de fusion pour une valeur de température spécifique.

**16.** Dispositif selon la revendication 15, **caractérisé en ce que** les descripteurs comprennent au moins 30 points de la dérivée seconde de chaque courbe de mesure.

**17.** Dispositif selon la revendication 15 ou 16, **caractérisé en ce que** les descripteurs comprennent en outre un ou plusieurs points de la dérivée première de chaque courbe de mesure et/ou un ou plusieurs points de chaque courbe de mesure.

**18.** Dispositif l'une quelconque des revendications 15 à 17, **caractérisé en ce que** les descripteurs comprennent en outre un ou plusieurs percentiles de chaque courbe de mesure.

**19.** Dispositif selon l'une quelconque des revendications 15 à 18, **caractérisé en ce que** les moyens (102) programmés pour la détermination (10) comprennent des moyens (102) programmés pour une détermination (10) par une méthode des forêts aléatoires.

**20.** Dispositif selon la revendication 19, **caractérisé en ce qu'**il comprend des moyens (101, 102) agencés et/ou programmés pour un apprentissage (6) comprenant :

- des moyens (101) agencés et/ou programmés pour une acquisition (1) de différentes courbes de fusion d'ADN, dites courbes de référence, à partir de différents échantillons biologiques de référence appartenant à différents groupes initiaux, puis
- des moyens (102) programmés pour une détermination (2, 3) des descripteurs à partir des courbes de référence, puis
- des moyens (102) programmés pour une construction (8) d'une forêt selon la méthode des forêts aléatoires, comprenant des moyens (102) programmés pour une construction de plusieurs arbres selon la méthode des forêts aléatoires, la ou les variable(s) étudiée(s) à chaque nœud de chaque arbre comprenant un ou plusieurs des descripteurs, chaque feuille de chaque arbre ne correspondant qu'à un seul groupe parmi les différents groupes possibles.

21. Dispositif selon la revendication 20, **caractérisé en ce que** les moyens (102) programmés pour la détermination (2, 3) des descripteurs comprennent :

   - des moyens (102) programmés pour une détermination préliminaire (2) de descripteurs,
   - des moyens (102) programmés pour, après la détermination préliminaire, une élimination (3) de certains descripteurs redondants.

22. Dispositif selon la revendication 21, **caractérisé en ce que** les moyens (102) programmés pour l'élimination (3) de certains descripteurs comprennent des moyens (102) programmés pour, pour chaque ensemble de descripteurs exhibant deux à deux un coefficient de corrélation de Pearson supérieur à 0,95, une conservation d'un seul descripteur.

23. Dispositif selon l'une quelconque des revendications 15 à 22, **caractérisé en ce qu'**il comprend :

   - des moyens (102) programmés pour, après l'acquisition de différentes courbes de référence, une identification de plusieurs courbes de référence correspondant au même groupe initial, dit groupe ambigu, et présentant des profils séparés en plusieurs sous-groupes, et
   - des moyens (102) programmés pour une séparation (4) de ce groupe ambigu en plusieurs groupes possibles.

24. Dispositif selon l'une quelconque des revendications 15 à 23, **caractérisé en ce qu'**il comprend :

   - des moyens (102) programmés pour, après l'acquisition de différentes courbes de référence, une identification de plusieurs courbes de référence correspondant à plusieurs groupes initiaux, dit groupes confondus, et présentant des profils réunis en un seul groupe, et
   - des moyens (102) programmés pour une unification (5) de ces groupes confondus en un unique groupe possible.

25. Dispositif selon l'une quelconque des revendications 15 à 24, **caractérisé en ce qu'**il comprend en outre des moyens (102) programmés pour un calcul d'un indice de confiance de l'étape de détermination d'une appartenance de l'échantillon biologique de mesure à un groupe déterminé.

26. Dispositif selon la revendication 25, **caractérisé en ce que** les moyens (102) programmés pour le calcul de l'indice de confiance comprennent :

   - des moyens (102) programmés pour un calcul d'une distribution de proximités moyennes entre courbes de référence appartenant au groupe déterminé,
   - des moyens (102) programmés pour un calcul d'une proximité moyenne de l'au moins une courbe de mesure avec les courbes de référence appartenant au groupe déterminé, et
   - des moyens (102) programmés pour un calcul d'un taux de courbes de référence appartenant au groupe déterminé, et ayant une proximité moyenne aux autres courbes de référence appartenant au groupe déterminé inférieure à la proximité moyenne de l'au moins une courbe de mesure avec les courbes de référence appartenant au groupe déterminé.

27. Dispositif selon la revendication 25 ou 26, **caractérisé en ce qu'**il comprend en outre des moyens (102) programmés pour, après l'étape de détermination d'une appartenance de l'échantillon biologique de mesure à un groupe déterminé, un refus d'affecter l'échantillon biologique de mesure à quelque groupe que ce soit en fonction de la valeur de l'indice de confiance.

**Patentansprüche**

1. Verfahren zur Klassifizierung einer biologischen Messprobe, umfassend:

- eine Erfassung (9) mindestens einer DNA-Schmelzkurve der biologischen Messprobe, mindestens eine Messkurve genannt,
- eine Bestimmung (10) einer Zugehörigkeit der biologischen Messprobe zu einer bestimmten Gruppe unter verschiedenen möglichen Gruppen durch Analyse von Deskriptoren, die aus der mindestens einen Messkurve hervorgehen,

wobei jeder der Erfassungs- und Bestimmungsschritte die Verwendung eines technischen Mittels (101, 102) beinhaltet,

**dadurch gekennzeichnet, dass** die Deskriptoren einen oder mehrere Punkte der zweiten Ableitung jeder Messkurve umfassen, wobei der oder jeder der Punkte einen Wert der zweiten Ableitung der Schmelzkurve für einen spezifischen Temperaturwert umfasst.

2. Verfahren nach Anspruch 1, **dadurch gekennzeichnet, dass** die Deskriptoren mindestens 30 Punkte der zweiten Ableitung jeder Messkurve umfassen.

3. Verfahren nach Anspruch 1 oder 2, **dadurch gekennzeichnet, dass** die Deskriptoren ferner einen oder mehrere Punkte der ersten Ableitung jeder Messkurve und/oder einen oder mehrere Punkte jeder Messkurve umfassen.

4. Verfahren nach einem der vorhergehenden Ansprüche, **dadurch gekennzeichnet, dass** die Deskriptoren ferner eine oder mehrere Perzentilen jeder Messkurve umfassen.

5. Verfahren nach einem der vorhergehenden Ansprüche, **dadurch gekennzeichnet, dass** die Bestimmung (10) eine Bestimmung durch ein Bootstrap-Forests-Verfahren umfasst.

6. Verfahren nach Anspruch 5, **dadurch gekennzeichnet, dass** es einen Lernprozess (6) umfasst, umfassend:

   - eine Erfassung (1) verschiedener DNA-Schmelzkurven, Referenzkurven genannt, auf Basis verschiedener biologischer Referenzproben, die verschiedenen Anfangsgruppen angehören, dann
   - eine Bestimmung (2, 3) der Deskriptoren auf Basis der Referenzkurven, dann
   - eine Konstruktion (8) eines Forests nach dem Random-Forest-Verfahren, umfassend eine Konstruktion mehrerer Bäume nach dem Random-Forest-Verfahren, wobei die studierte(n) Variable(n) an jedem Knoten jedes Baums einen oder mehrere Deskriptoren umfassen, wobei jedes Blatt jedes Baums nur einer einzigen Gruppe unter den verschiedenen möglichen Gruppen entspricht.

7. Verfahren nach Anspruch 6, **dadurch gekennzeichnet, dass** die Bestimmung (2, 3) der Deskriptoren umfasst:

   - eine vorherige Bestimmung (2) von Deskriptoren, dann
   - eine Beseitigung (3) von redundanten Deskriptoren.

8. Verfahren nach Anspruch 7, **dadurch gekennzeichnet, dass** die Beseitigung (3) gewisser Deskriptoren für jede Gesamtheit von Deskriptoren, die zwei und zwei einen Pearson-Korrelationskoeffizienten über 0,95 zeigen, eine Beibehaltung eines einzigen Deskriptors umfasst.

9. Verfahren nach einem der vorhergehenden Ansprüche, **dadurch gekennzeichnet, dass** es umfasst:

   - nach der Erfassung verschiedener Referenzkurven eine Identifikation mehrerer Referenzkurven entsprechend der gleichen Anfangsgruppe, mehrdeutige Gruppe genannt, die in mehrere Untergruppen getrennte Profile aufweist, und
   - eine Trennung (4) dieser mehrdeutigen Gruppe in mehrere mögliche Gruppen.

10. Verfahren nach einem der vorhergehenden Ansprüche, **dadurch gekennzeichnet, dass** es umfasst:

    - nach der Erfassung verschiedener Referenzkurven eine Identifikation mehrerer Referenzkurven entsprechend mehreren Anfangsgruppen, zusammenfallende Gruppen genannt, die zu einer Gruppe vereinte Profile aufweisen, und
    - eine Vereinigung (5) dieser zusammenfallenden Gruppen zu einer einzigen möglichen Gruppe.

11. Verfahren nach einem der vorhergehenden Ansprüche, **dadurch gekennzeichnet, dass** es ferner eine Berechnung eines Vertrauensindex des Schritts der Bestimmung einer Zugehörigkeit der biologischen Messprobe zu einer bestimmten Gruppe umfasst.

12. Verfahren nach Anspruch 11, **dadurch gekennzeichnet, dass** die Berechnung des Vertrauensindex umfasst:

- eine Berechnung einer Verteilung von durchschnittlichen Nähen zwischen der bestimmten Gruppe angehörenden Referenzkurven,
- eine Berechnung einer durchschnittlichen Nähe der mindestens einen Messkurve mit den der bestimmten Gruppe angehörenden Referenzkurven, und
- eine Berechnung einer Rate von der bestimmten Gruppe angehörenden Referenzkurven, die eine durchschnittliche Nähe zu den anderen der bestimmten Referenzgruppe angehörenden Kurven haben, die kleiner als die durchschnittliche Nähe der mindestens einen Messkurve mit den der bestimmten Gruppe angehörenden Referenzkurven ist.

13. Verfahren nach Anspruch 11 oder 12, **dadurch gekennzeichnet, dass** es ferner nach dem Schritt der Bestimmung einer Zugehörigkeit der biologischen Messprobe zu einer bestimmten Gruppe eine Verweigerung der Zuordnung der biologischen Messprobe zu irgendeiner Gruppe in Abhängigkeit vom Wert des Vertrauensindex umfasst.

14. Verfahren nach einem der vorhergehenden Ansprüche, **dadurch gekennzeichnet, dass** die Erfassung mindestens einer DNA-Schmelzkurve der biologischen Messprobe die Erfassung mindestens einer Schmelzkurve eines Resultats einer PCR umfasst, das bei gleichzeitigem Vorhandensein mehrerer Paare von Ansätzen, die mehrere DNA-Zielmoleküle anvisieren, erhalten wird.

15. Vorrichtung (100) zur Klassifizierung einer biologischen Messprobe, umfassend:

- Mittel (101), die für eine Erfassung (9) mindestens einer DNA-Schmelzkurve der biologischen Messprobe, mindestens eine Messkurve genannt, vorgesehen und/oder programmiert sind,
- Mittel (102), die für eine Bestimmung (10) einer Zugehörigkeit der biologischen Messprobe zu einer bestimmten Gruppe unter verschiedenen möglichen Gruppen durch Analyse von Deskriptoren, die aus der mindestens einen Messkurve hervorgehen, programmiert sind,

**dadurch gekennzeichnet, dass** die Deskriptoren einen oder mehrere Punkte der zweiten Ableitung jeder Messkurve umfassen, wobei der oder jeder der Punkte einen Wert der zweiten Ableitung der Schmelzkurve für einen spezifischen Temperaturwert umfasst.

16. Vorrichtung nach Anspruch 15, **dadurch gekennzeichnet, dass** die Deskriptoren mindestens 30 Punkte der zweiten Ableitung jeder Messkurve umfassen.

17. Vorrichtung nach Anspruch 15 oder 16, **dadurch gekennzeichnet, dass** die Deskriptoren ferner einen oder mehrere Punkte der ersten Ableitung jeder Messkurve und/oder einen oder mehrere Punkte jeder Messkurve umfassen.

18. Vorrichtung nach einem der Ansprüche 15 bis 17, **dadurch gekennzeichnet, dass** die Deskriptoren ferner eine oder mehrere Perzentilen jeder Messkurve umfassen.

19. Vorrichtung nach einem der Ansprüche 15 bis 18, **dadurch gekennzeichnet, dass** die für die Bestimmung (10) programmierten Mittel (102) Mittel (102) umfassen, die für eine Bestimmung (10) durch ein Random-Forest-Verfahren programmiert sind.

20. Vorrichtung nach Anspruch 19, **dadurch gekennzeichnet, dass** sie Mittel (101, 102) umfasst, die für einen Lernprozess (6) vorgesehen und/oder programmiert sind, umfassend:

- Mittel (101), die für eine Erfassung (1) verschiedener DNA-Schmelzkurven, Referenzkurven genannt, auf Basis verschiedener biologischer Referenzproben, die verschiedenen Anfangsgruppen angehören, vorgesehen und/oder programmiert sind, dann
- Mittel (102), die für eine Bestimmung (2, 3) der Deskriptoren auf Basis der Referenzkurve programmiert sind, dann
- Mittel (102), die für eine Konstruktion (8) eines Forests nach dem Bootstrap-Forests-Verfahren programmiert sind, umfassend die Mittel (102), die für eine Konstruktion mehrerer Bäume nach dem Radom-Forest-Verfahren programmiert sind, wobei die studierte(n) Variable(n) an jedem Knoten jedes Baums einen oder mehrere Deskriptoren umfassen, wobei jedes Blatt jedes Baums nur einer einzigen Gruppe unter den verschiedenen möglichen Gruppen entspricht.

**21.** Vorrichtung nach Anspruch 20, **dadurch gekennzeichnet, dass** die Mittel (102), die für die Bestimmung (2, 3) der Deskriptoren programmiert sind, umfassen:

- Mittel (102), die für eine vorherige Bestimmung (2) von Deskriptoren programmiert sind,
- Mittel (102), die für eine Beseitigung (3), nach der vorherigen Bestimmung, gewisser redundanten Deskriptoren programmiert sind.

**22.** Vorrichtung nach Anspruch 21, **dadurch gekennzeichnet, dass** die Mittel (102), die für die Beseitigung (3) gewisser Deskriptoren programmiert sind, Mittel (102) umfassen, die für jede Gesamtheit von Deskriptoren, die zwei und zwei einen Pearson-Korrelationskoeffizienten über 0,95 zeigen, für eine Beibehaltung eines einzigen Deskriptors programmiert sind.

**23.** Vorrichtung nach einem der Ansprüche 15 bis 22, **dadurch gekennzeichnet, dass** sie umfasst:

- Mittel (102), die nach der Erfassung verschiedener Referenzkurven für eine Identifikation mehrerer Referenzkurven entsprechend der gleichen Anfangsgruppe, mehrdeutige Gruppe genannt, die in mehrere Untergruppen getrennte Profile aufweist, programmiert sind, und
- Mittel (102), die für eine Trennung (4) dieser mehrdeutigen Gruppe in mehrere mögliche Gruppen programmiert sind.

**24.** Vorrichtung nach einem der Ansprüche 15 bis 23, **dadurch gekennzeichnet, dass** sie umfasst:

- Mittel (102), die nach der Erfassung verschiedener Referenzkurven für eine Identifikation mehrerer Referenzkurven entsprechend mehreren Anfangsgruppen, zusammenfallende Gruppen genannt, die zu einer Gruppe vereinte Profile aufweisen, programmiert sind, und
- Mittel (102), die für eine Vereinigung (5) dieser zusammenfallenden Gruppen zu einer möglichen einzigen Gruppe programmiert sind.

**25.** Vorrichtung nach einem der Ansprüche 15 bis 24, **dadurch gekennzeichnet, dass** sie ferner Mittel (102) umfasst, die für eine Berechnung eines Vertrauensindex des Schritts der Bestimmung einer Zugehörigkeit der biologischen Messprobe zu einer bestimmten Gruppe programmiert sind.

**26.** Vorrichtung nach Anspruch 25, **dadurch gekennzeichnet, dass** die Mittel (102), die für die Berechnung des Vertrauensindex programmiert sind, umfassen:

- Mittel (102), die für eine Berechnung einer Verteilung von durchschnittlichen Nähen zwischen der bestimmten Gruppe angehörenden Referenzkurven programmiert sind,
- Mittel (102), die für eine Berechnung einer durchschnittlichen Nähe der mindestens einen Messkurve mit den der bestimmten Gruppe angehörenden Referenzkurven programmiert sind, und
- Mittel (102), die für eine Berechnung einer Rate von der bestimmten Gruppe angehörenden Referenzkurven, die eine durchschnittliche Nähe zu den anderen der bestimmten Referenzgruppe angehörenden Kurven haben, die kleiner als die durchschnittliche Nähe der mindestens einen Messkurve mit den der bestimmten Gruppe angehörenden Referenzkurven ist, programmiert sind.

**27.** Vorrichtung nach Anspruch 25 oder 26, **dadurch gekennzeichnet, dass** sie ferner Mittel (102) umfasst, die nach dem Schritt der Bestimmung einer Zugehörigkeit der biologischen Messprobe zu einer bestimmten Gruppe für eine Verweigerung der Zuordnung der biologischen Messprobe zu irgendeiner Gruppe in Abhängigkeit vom Wert des Vertrauensindex programmiert sind.

**Claims**

**1.** Method for classifying a measurement biological sample, comprising:

- acquisition (9) of at least one DNA melting curve of the measurement biological sample, called at least one measurement curve,
- determination (10) that the measurement biological sample belongs to a defined group among different possible groups, by analysis of descriptors originating from the at least one measurement curve,

each one of the acquisition step and determination step involving the use of a technical mean (101, 102), **characterized in that** the descriptors comprise one or more points of the second derivative of each measurement curve, said or each of said points comprising a value of the second derivative of the melting curve for a specific temperature value.

2. Method according to claim 1, **characterized in that** the descriptors comprise at least 30 points of the second derivative of each measurement curve.

3. Method according to claim 1 or 2, **characterized in that** the descriptors further comprise one or more points of the first derivative of each measurement curve and / or one or more points of each measurement curve.

4. Method according to any one of the preceding claims, **characterized in that** the descriptors further comprise one or more percentiles of each measurement curve.

5. Method according to any one of the preceding claims, **characterized in that** the determination (10) comprises determination by a random forest method.

6. Method according to claim 5, **characterized in that** it comprises learning (6) comprising:

- acquisition (1) of different DNA melting curves, called reference curves, starting from different reference biological samples belonging to different initial groups, then
- determination (2, 3) of the descriptors starting from the reference curves, and then
- construction (8) of a forest by the random forest method, comprising construction of several trees by the random forest method, the variable or variables studied at each node of each tree comprising one or more of the descriptors, each leaf of each tree only corresponding to a single group among the different possible groups.

7. Method according to claim 6, **characterized in that** determination (2, 3) of the descriptors comprises:

- a preliminary determination (2) of descriptors, and then
- an elimination (3) of certain redundant descriptors.

8. Method according to claim 7, **characterized in that** the elimination (3) of certain descriptors comprises, for each set of descriptors displaying in pairs a Pearson correlation coefficient greater than 0.95, retention of a single descriptor.

9. Method according to any one of the preceding claims, **characterized in that** it comprises:

- after acquisition of different reference curves, identification of several reference curves corresponding to the same initial group, called ambiguous group, and having profiles separated into several subgroups, and
- separation (4) of this ambiguous group into several possible groups.

10. Method according to any one of the preceding claims, **characterized in that** it comprises:

- after acquisition of different reference curves, identification of several reference curves corresponding to several initial groups, called merged groups, and having profiles combined in a single group, and
- unification (5) of these merged groups into a single possible group.

11. Method according to any one of the preceding claims, **characterized in that** it further comprises calculation of a confidence index of the step of determining that the measurement biological sample belongs to a defined group.

12. Method according to claim 11, **characterized in that** calculation of the confidence index comprises:

- calculation of a distribution of mean proximities between reference curves belonging to the defined group,

- calculation of a mean proximity of the at least one measurement curve with the reference curves belonging to the defined group, and
- calculation of a level of reference curves belonging to the defined group, and having a mean proximity to the other reference curves belonging to the defined group less than the mean proximity of the at least one measurement curve with the reference curves belonging to the defined group.

13. Method according to claim 11 or 12, **characterized in that** it further comprises, after the step of determining that the measurement biological sample belongs to a defined group, a refusal to assign the measurement biological sample to any group whatever, as a function of the value of the confidence index.

14. Method according to any one of the preceding claims, **characterized in that** the acquisition of at least one DNA melting curve of the measurement biological sample comprises acquisition of at least one melting curve of a result of a PCR obtained in the simultaneous presence of several primer pairs targeting several target DNA molecules.

15. Device (100) for classifying a measurement biological sample, comprising:

- means (101) arranged and/or programmed for acquisition (9) of at least one DNA melting curve of the measurement biological sample, called at least one measurement curve,
- means (102) programmed for determining (10) that the measurement biological sample belongs to a defined group among different possible groups, by analysis of descriptors originating from the at least one measurement curve,

**characterized in that** the descriptors comprise one or more points of the second derivative of each measurement curve, said or each of said points comprising a value of the second derivative of the melting curve for a specific temperature value.

16. Device according to claim 15, **characterized in that** the descriptors comprise at least 30 points of the second derivative of each measurement curve.

17. Device according to claim 15 or 16, **characterized in that** the descriptors further comprise one or more points of the first derivative of each measurement curve and / or one or more points of each measurement curve.

18. Device according to any one of claims 15 to 17, **characterized in that** the descriptors further comprise one or more percentiles of each measurement curve.

19. Device according to any one of claims 15 to 18, **characterized in that** the means (102) programmed for determination (10) comprise means (102) programmed for determination (10) by a random forest method.

20. Device according to claim 19, **characterized in that** it comprises means (101, 102) arranged and/or programmed for learning (6) comprising:

- means (101) arranged and/or programmed for acquisition (1) of different DNA melting curves, called reference curves, starting from different reference biological samples belonging to different initial groups, then
- means (102) programmed for determination (2, 3) of the descriptors from the reference curves, then
- means (102) programmed for construction (8) of a forest by the random forest method, comprising means (102) programmed for construction of several trees by the random forest method, the variable or variables studied at each node of each tree comprising one or more of the descriptors, each leaf of each tree only corresponding to a single group among the different possible groups.

21. Device according to claim 20, **characterized in that** the means (102) programmed for determination (2, 3) of the descriptors comprise:

- means (102) programmed for the preliminary determination (2) of descriptors,
- means (102) programmed for, after the preliminary determination,

an elimination (3) of certain redundant descriptors.

22. Device according to claim 21, **characterized in that** the means (102) programmed for the elimination (3) of certain descriptors comprise means (102) programmed for, for each set of descriptors displaying in pairs a Pearson correlation coefficient greater than 0.95, retention of a single descriptor.

23. Device according to any one of claims 15 to 22, **characterized in that** it comprises:

- means (102) programmed for, after acquisition of different reference curves, identification of several reference curves corresponding to the same initial group, called ambiguous group, and having profiles separated into several subgroups, and
- means (102) programmed for separation (4) of this ambiguous group into several possible groups.

24. Device according to any one of claims 15 to 23, **characterized in that** it comprises:

- means (102) programmed for, after acquisition of different reference curves, identification of several reference curves corresponding to several initial groups, called merged groups, and having profiles combined in a single group, and
- means (102) programmed for unification (5) of these merged groups into a single possible group.

25. Device according to any one of claims 15 to 24, **characterized in that** it further comprises means (102) programmed for calculating a confidence index of the step of determining that the measurement biological sample belongs to a defined group.

26. Device according to claim 25, **characterized in that** the means (102) programmed for calculating the confidence index comprise:

- means (102) programmed for calculating a distribution of mean proximities between reference curves belonging to the defined group,
- means (102) programmed for calculating a mean proximity of the at least one measurement curve with the reference curves belonging to the defined group, and
- means (102) programmed for calculating a level of reference curves belonging to the defined group, and having a mean proximity to the other reference curves belonging to the defined group less than the mean proximity of the at least one measurement curve with the reference curves belonging to the defined group.

27. Device according to claim 25 or 26, **characterized in that** it further comprises means (102) programmed for, after the step of determining that the measurement biological sample belongs to a defined group, a refusal to assign the measurement biological sample to any group whatever, as a function of the value of the confidence index.

FIG. 1

FIG. 2

FIG. 9

FIG. 3

FIG. 4

FIG. 5

FIG. 6

FIG. 7

FIG. 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2013166373 A

**Littérature non-brevet citée dans la description**

- **DE RÖDIGER et al.** Surface Melting Curve Analysis with R. *The R Journal,* 02 Décembre 2013, vol. 5
- **LEO BREIMAN.** Random Forests. *Machine learning,* 2001, vol. 45, 5-32
- **ANDY LIAW ; MATTHEW WIENER.** Classification and Régression by RandomForest. *R News,* 03 Décembre 2002, vol. 2, ISSN 1609-3631
- **LEO BREIMAN.** Manual on setting up, using, and understanding random Forests V3.1. 2002